# EUROPÄISCHE PATENTSCHRIFT

(11) **EP 2 594 188 B1**
(45) Veröffentlichungstag und Bekanntmachung des Hinweises auf die Patenterteilung: **16.08.2017**
(21) Anmeldenummer: 12192614.1
(22) Anmeldetag: 14.11.2012
(51) Int. Cl.: A61B 1/01, A61B 1/12, A61B 1/015, A61B 1/05, A61B 1/06

(54) **Medizinisches Endoskop mit einer Kühlvorrichtung**
Medical endoscope comprising cooling means
Endoscope avec un dispositif de refroisissement

(30) Priorität: 18.11.2011 DE 102011055526
(43) Veröffentlichungstag der Anmeldung: 22.05.2013
(73) Patentinhaber: invendo medical GmbH, 69469 Weinheim (DE)
(72) Erfinder: Dillinger, Anja Katrin, 86447 Aindling (DE); Schütz, Günter Wilhelm, 86152 Augsburg (DE); Dr. Schnürer-Patschan, Christoph, 85456 Wartenberg (DE)
(74) Vertreter: Winter, Brandl, Fürniss, Hübner, Röss, Kaiser, Polte - Partnerschaft mbB

(56) Entgegenhaltungen:
- EP-A2- 1 421 894
- WO-A2-2007/133594
- DE-A1-102005 030 861
- JP-A- 2002 177 197
- US-A1- 2007 249 907

## Beschreibung

Die vorliegende Erfindung betrifft ein medizinisches Endoskop, in dessen Endoskopkopf elektrische Bauelemente eingebaut sind, die mittels einer internen Kühlvorrichtung gekühlt werden und insbesondere ein Endoskop gemäß dem Oberbegriff des Patentanspruch 1.

Elektronische/elektrische Bauelemente wie lichtempfindliche Sensoren (CMOS-Chips) lichtemittierende Dioden (LED's), usw., werden in zunehmendem Maß in medizinischen Vorrichtungen, beispielsweise Endoskopievorrichtungen, eingesetzt. Insbesondere Endoskope/Koloskope haben an ihrem distalen Ende eines flexiblen Endoskopschafts einen sogenannten Endoskopkopf, in dem eine Anzahl von Funktionseinheiten wie u.a. eine optische Einrichtung bestehend aus einem Linsensystem und lichtempfindlichem Mikrochip, eine Beleuchtungseinrichtung, vorzugsweise ein Arbeitskanal sowie ggf. eine Spülvorrichtung untergebracht sind. Aufgrund des herrschenden Platzmangels innerhalb des Endoskopkopfs sind die einzelnen Funktionseinheiten sehr eng aneinander gebaut, derart, dass sie sich ggf. thermisch gegenseitig beeinflussen. In anderen Worten ausgedrückt entwickelt insbesondere die Beleuchtungseinrichtung eine thermische Energie, die nicht ausschließlich über die Außenfläche des Endoskopkopfs abgeführt werden darf, um das anliegende biologische Gewebe (Patientendarm, Magen, Speiseröhre, etc.) nicht zu schädigen. Diese thermische Energie führt ferner zu Beeinträchtigungen der optischen Einrichtung insbesondere des in der Regel wärmeempfindlichen Lichtsensors. Aus diesem Grund werden thermische Energie abstrahlende und/oder wärmeempfindliche Funktionseinheiten mittels einer integrierten Kühlvorrichtung zusätzlich aktiv gekühlt.

Benachbarter Stand der Technik ist aus den Druckschriften US 2007/0249907 A1, JP2002-177197A, DE 10 2005 030 861 A1, WO 2007/133594 A2 und EP 1 421 894 A2 bekannt.

Aus der US 7,413,543 B2 ist ein medizinisches Endoskop der einschlägigen Gattung bekannt. Der Endoskopkopf dieses bekannten Endoskops wird durch eine hülsenförmige sowie stirnseitig mit einer einstückig daran ausgeformten Endplatte verschlossene Endoskopkappe ausgebildet, in die ein Linsensystem, eine elektronische Steuerung mit lichtempfindlichem Sensorchip, eine elektrische Leiterplatte mit darauf angebrachten LED's sowie eine Kühlvorrichtung als Funktionseinheiten eingebaut sind. Darüber hinaus wird der Endoskopkopf von einem Arbeitskanal axial durchzogen, der an der Endplatte nach Außen mündet.

Die Kühlvorrichtung besteht aus einem separaten Wärmetauscher in der Form einer halbzylindrischen Kühlkammermanschette mit einem Fluidein- und -auslass, der in die Endoskopkappe eingesteckt ist. Die distale offene Stirnseite der Kühlkammer ist durch die elektrische Leiterplatte verschlossen, derart, dass Kühlfluid innerhalb der Kammer unmittelbar die Leiterplatte anströmt und somit die darauf montierten LED's rückseitig kühlt. Gleichzeitig dient die Kühlkammer als Halterung für das Linsensystem. In der Endplatte sind Durchgangsbohrungen ausgebildet, sodass die LED's nach einem Einstecken der Kühlkammer in die Endoskopkappe Licht durch die Bohrungen nach Außen abgeben können. An der proximalen, geschlossenen Stirnseite der Kühlkammer ist die elektronische Steuerung mit lichtempfindlichem Sensorchip angeordnet, sodass auch der Sensor von der Kühlkammer etwas gekühlt wird. Gleichzeitig dient die Kühlkammer als Wärme abschirmender Abstandshalter, der die LED's vom lichtempfindlichen Sensor axial beabstandet, um die gegenseitige thermische Beeinflussung möglichst klein zu halten.

Gemäß der US 7,413,543 B2 bildet die Kühlkammermanschette und die Leiterplatte mit den darauf montierten LED's eine separate, eigenständige Funktionseinheit, die außerhalb der Endoskopkappe vormontiert und dann in die Kappe eingeschoben/eingesteckt wird.

Demzufolge müssen in der Endoskopkappe Halterungen für die Kühlkammer ausgebildet sein. Diese schränken das ohnehin nur begrenzte Platzangebot weiter ein und verteuern die Herstellung der Endoskopkappe.

Angesichts dieser Problematik ist es die Aufgabe der vorliegenden Erfindung den Endoskopkopf eines vorzugsweise medizinischen Endoskops so weiterzubilden, dass dieser eine höhere Effizienz erreicht. Ein Ziel ist es, die Kosten für dessen Herstellung zu minimieren. Schließlich ist es ein Ziel der Erfindung, durch geeignete Anordnung und Zuordnung der einzelnen Funktionseinheiten mehr Freiraum zu erhalten, die für zusätzliche Funktionseinheiten oder eine Vergrößerung einzelner Funktionseinheiten ausgenutzt werden kann.

Die vorstehende Aufgabe wird durch ein Endoskop mit Endoskopkopf gemäß dem Patentanspruch 1 gelöst. Vorteilhafte Ausgestaltungen der Erfindung sind u.a. Gegenstand der Unteransprüche.

Der Grundgedanke der Erfindung beruht darauf, möglichst viele oder besonders ausgewählte Funktionseinheiten zu einem integralen Bauteil zusammenzufassen, um so Bauraum für Halte- und Führungseinrichtungen für den Zusammenbau der einzelnen Funktionseinheiten einzusparen, wobei der eingesparte Bauraum dann anderweitig, beispielsweise für voluminösere Leuchtmittel, einen Arbeitskanal mit vergrößertem Durchmesser oder dergleichen genutzt werden kann. Ein Nebeneffekt dieser integralen bzw. einstückigen Bauweise ist es, dass einzelne Funktionseinheiten wie etwa die Beleuchtung ggf. weiter in Richtung hin zur distalen Stirnseite des Endoskopkopfs vorgeschoben werden können, sodass deren Effizienz ggf. verbessert wird, ohne dass deren Leistung erhöht werden muss.

Im Konkreten ist folglich ein Endoskop gemäß einem Aspekt der Erfindung vorgesehen, mit einem flexiblen Endoskopschaft, an dessen distalem Ende ein Endoskopkopf angeordnet ist, bestehend aus einer vorzugsweise an beiden Stirnseiten offenen Hülse, in der Funktionseinheiten, insbesondere eine Optik, ein Arbeitskanal, eine Beleuchtung und eine Kühlung für die Beleuchtung untergebracht sind. Die Kühlung umfasst einen in der Hülse einstückig mit der Hülse ausgeformten Wärmetauscher, der innerhalb der Hülse eine geschlossene Kühlkammer zur Erzeugung einer Kühlmittelströmung (im Wesentlichen einstückig mit der Hülse) ausbildet. Die Beleuchtung in Form einer ein oder mehrere Leuchtmittel tragenden externen Leiterplatte ist so in der Hülse platziert, dass sie an der distalen Stirnwand des Wärmetauschers anliegt, um eine thermische Energie über die Stirnwand des Wärmetauschers in die Kühlmittelströmung abzugeben.

Die Endoskopkopfhülse und der Wärmetauscher, bzw. die Kühlkammer bilden somit eine in einem einzigen Herstellungsvorgang herstellbare Einheit vorzugsweise aus dem gleichen Material. Dies trägt deutlich zur Verringerung der Herstellungskosten bei. Darüber hinaus entfallen weitere Halterungen etwa für eine separat hergestellte Kühlkammer (Wärmetauscher), sodass auch Platz eingespart werden kann. Ein Vorteil ist es auch, wenn die Kühlkammer unmittelbar an die proximale Rückseite der Beleuchtung insbesondere in Form der Leiterplatte angrenzt, derart, dass die Beleuchtung insbesondere in Form der Leiterplatte an der Kühlkammer anliegt. Auf diese Weise wird die Übertragung von thermaler Energie in das Kühlmittel innerhalb der Kühlkammer verbessert und die Effizienz der Kühlung (d.h. des Wärmetauschers) erhöht. Gleichzeitig ist die Beleuchtung vor einer unmittelbaren Beaufschlagung durch das Kühlmittel geschützt, sodass die Beleuchtung beispielsweise durch eine konventionelle LED-Platine bereitgestellt werden kann, die keine Isolationseigenschaften zum Schutz gegen Feuchtigkeit/Nässe aufweisen muss. Dies trägt ebenfalls zur Kostenreduktion bei.

Grundsätzlich dient eine Platine/Leiterplatte dazu, elektrische Elemente wie die Leuchtmittel zu tragen und elektrisch zu verbinden. Gemäß dem Stand der Technik können derartige Leiterplatten jedoch zusätzlich auch mit einer (aussteifenden) Trägerplatte vorzugsweise aus Aluminium in elektrisch isolierender Weise (durch Bonden oder Kleben) verbunden sein, um eine höhere Wärmeabstrahlung bei erhöhter mechanischer Festigkeit zu erreichen. Im Fall der vorliegenden Erfindung ist es vorteilhaft, eine solche (Verbund-) Trägerplatte mit erhöhter Wärmeabstrahlungskapazität zu verwenden, um die Übertragung von thermischer Energie über die axiale Stirnwand des Wärmetauschers/Kühlkammer in das Kühlmittel zu verbessern.

Vorteilhaft ist es, wenn in der Hülse im axialen Bereich ihrer distalen Stirnseite eine radial nach innen ragende, sowie einstückig am Wärmetauscher sich abstützende Halterung für den Arbeitskanal und vorzugsweise für einen Spülkanal angeformt ist, wobei die Halterung ferner eine axiale Durchgangsöffnung für die Optik aufweist. Dies bedeutet, dass der Wärmetauscher (erste Funktionseinheit), die Hülse (zweite Funktionseinheit) zum Schutz der elektrischen/optischen Einrichtungen sowie die Halterungen (dritte Funktionseinheit) für den Arbeits- und ggf. Spülkanal aus einem gemeinsamen Bauteil gefertigt sind und sich gegenseitig stabilisieren. Im Fall, dass dieses Bauteil ein Spritzguss- oder Pressbauteil vorzugsweise aus Kunststoff ist, kann dieses besonders preisgünstig hergestellt werden.

Ein anderer ggf. unabhängiger Aspekt der Erfindung sieht vor, dass die Kühlkammer einen nach Außerhalb der Kühlkammer abgeschlossenen Kühlmittelpfad vorzugsweise in Form eines in der Kühlkammer definierten Kanals ausbildet, der zumindest abschnittsweise entlang der distalen Stirnwand des Wärmetauschers verläuft und weiter vorzugsweise zur Erhöhung der Kanalstrecke eine Anzahl von Kanalschleifen ausbildet. In anderen Worten ausgedrückt betrifft ein weiterer (unabhängiger oder kombinierbarer) Aspekt der Erfindung ein nach außen hin fluiddichtes, d.h. geschlossenes Kanalsystem innerhalb der Kühlkammer mit einem Kühlmittelein- und -auslass, das zumindest abschnittsweise unmittelbar an der distalen Stirnwand des Wärmetauschers/Kühlkammer vorbeigeführt ist. D.h., in diesem Fall wird innerhalb der Kühlkammer eine Art Rohr- oder Kanalsystem ausgebildet/eingesetzt, durch das Kühlmittel in vorbestimmten Bahnen vergleichbar zu einem bekannten Wärmetauscher geführt wird, wobei die Kanalwände selbst die vorstehend genannte Stirnwand bilden oder die Kanalwände an der Stirnwand anliegen.

Vorzugsweise wird demnach das Kanalsystem durch eine (spiral- oder schneckförmig sich erstreckenden) Rohrleitung gebildet, die separat gefertigt und in die Kühlkammer eingesetzt ist oder das Kanalsystem ist einstückig mit der Kühlkammer und damit mit der Endoskopkopfhülse (vorzugsweise in einem einzigen Herstellungsvorgang) ausgeformt.

Ein weiterer ggf. unabhängiger Aspekt der Erfindung sieht eine externe (separat hergestellte) Trennwand vor, welche die distale Stirnwand des Wärmetauschers zum stirnseitig dichtenden Verschließen und fluiddichten Trennen der Kühlkammer von der Beleuchtung sowie zur Gewährleistung einer Übertragung an thermischer Energie von der Beleuchtung in das Kühlmittel ausbildet. Dies hat den Vorteil, dass die distale Stirnwand des Wärmetauschers mit geeigneten thermischen und mechanischen Eigenschaften ausgestattet sein kann, wie beispielsweise einen gegenüber dem Hülsenmaterial erhöhten Wärmeübergangskoeffizienten. Dies ist besonders dann wichtig, wenn die Hülse zum Schutz des umgebenden Organgewebes eines Patienten eher wärmeisolierend wirkt. Auch kann die Trennwand aus einem besonders flexiblen oder elastischen Material gefertigt sein, das sich der Struktur an der Unterseite der Leuchtmittel tragenden Platine anpasst und so den Wärmeübergang verbessert. Beispielsweise kann es vorgesehen sein, dass die Trennwand eine Membran oder Folie ist, die mit der Hülse fluiddicht verklebt und/oder vergossen ist.

Ein weiterer, ggf. unabhängiger Aspekt der Erfindung betrifft die Form der distalen Stirnwand des Wärmetauschers/Kühlkammer in Draufsicht insbesondere dann, wenn diese als Trennwand separat zur Hülse ausgebildet ist. In diesem Fall ist die Trennwand unterschiedlich zu der Form der die Leuchtmittel tragenden externen Leiterplatte nur an die Form der Kühlkammer angepasst ist, um ausschließlich die Kühlkammer axial abzudecken. Die Leiterplatte/Platine hat nämlich eine Vielzahl von elektrischen Elementen einschließlich elektrischer Anschlüsse/Kontakte, die am Wärmetauscher/Kühlkammer vorbeigeführt werden müssen. Wäre daher die Trennplatte an die Form der Leiterplatte angepasst, müsste die Trennplatte mit entsprechenden Durchgangsöffnungen versehen sein, durch welche die Kontakte geführt sind. Im Prinzip wäre dann die Trennwand ein weiterer Bestandteil der Leiterplatte/Platine.

Erfindungsgemäß aber ist die Trennwand nur der Kühlkammer angepasst, ist also Bestandteil des Wärmetauschers und nicht der Leiterplatte. In diesem Fall können die Kontakte der Leiterplatte auch an der Trennwand vorbeigeführt werden. Dies erspart eine Bearbeitung der Trennwand zu deren Anpassung an die Leiterplatte, wodurch sich der Herstellungsvorgang vereinfacht.

Ferner sieht ein weiterer ggf. unabhängiger Aspekt der Erfindung vor, dass die Optik aus einer einen lichtempfindlichen Sensorchip tragenden Leiterplatte besteht, auf der oberhalb des Chips ein Linsensystem montiert ist, derart, dass das Linsensystem kontaktlos zur Hülse ausschließlich über die Sensorchip tragende Leiterplatte gehalten und/oder ausgerichtet wird und frei durch die Durchgangsöffnung in der Halterung für den Arbeitskanal geführt ist. Die Halterung bzw. die Hülse dient somit nicht zur Ausrichtung der Optik und kann daher auch mit Toleranzen einfach hergestellt werden.

Schließlich betrifft ein anderer, ggf. unabhängiger Aspekt der Erfindung die distale Stirnseite der Hülse. Demzufolge ist es vorgesehen, dass der axiale Abschnitt zwischen der Leuchtmittel tragenden, externen Leiterplatte und der distalen Stirnseite der Hülse mit einer lichtdurchlässigen Vergussmasse aufgefüllt ist, welche stirnseitig entweder mittels einer transparenten Platte abgedeckt ist oder welche zusammen mit der distalen Vorderseite der Halterung für den Arbeitskanal die axiale Stirnseite der Hülse bildet.

In anderen Worten ausgedückt ist gemäß einem Aspekt der Erfindung die Beleuchtung quasi in die Stirnseite der Hülse integriert. D. h. die Hülse dient dann nicht nur als Schutzkappe für die darin eingebauten Funktionseinheiten sondern ist auch mit einer entsprechenden Ausnehmung an deren distaler Stirnseite versehen oder an der distalen Stirnseite im Wesentlichen offen, sodass die Beleuchtung von der distalen Seite her in die Hülse eingesetzt und darin fixiert werden kann. Dies hat den Vorteil, dass eine zusätzliche Halterung für die Beleuchtung entfällt.

Je nach dem, wie weit die Beleuchtung zur distalen Stirnseite der Hülse vorgeschoben ist, bildet diese insbesondere im Fall einer Leuchtmittel tragenden Leiterplatte einen Bestandteil der distalen Endplatte zum axialen Verschließen der Hülse, wobei weiter vorzugsweise eine lichtdurchlässige Platte über die Leiterplatte gelegt ist, um die elektrischen Kontakte und Leiterbahnen beispielsweise vor Feuchtigkeit zu schützen. Die lichtdurchlässige Platte kann entweder direkt auf die Leuchtmittel tragende Leiterplatte (Platine) oder auf die Endplatte aufgesetzt sein. Je nach Wandstärke der Endplatte kann die lichtdurchlässige Platte auch in der speziellen Durchgangsöffnung eingesetzt sein.

Die Erfindung wird nachstehend anhand eines bevorzugten Ausführungsbeispiels unter Bezugnahme auf die begleitenden Zeichnung näher erläutert.

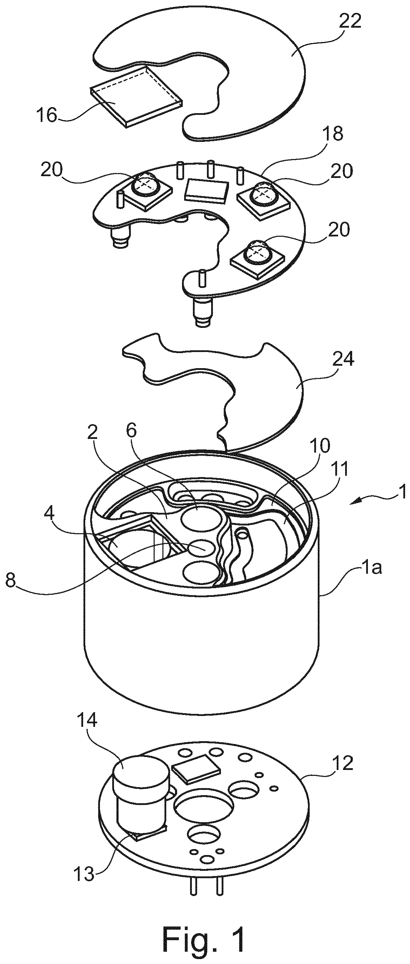

Fig. 1 zeigt die Explosionsdarstellung eines Endoskopkopfs für ein flexibles medizinisches Endoskop gemäß einem bevorzugten Ausführungsbeispiel der Erfindung.

Gemäß der anliegenden Figur 1 hat ein Endoskopkopf nach einem bevorzugten Ausführungsbeispiel der Erfindung eine zylinderförmige Endoskopkopfhülse (nachfolgend einfach als Hülse bezeichnet) 1, welche an das distale Ende eines nicht weiter dargestellten, vorzugsweise flexiblen Endoskopschafts (Koloskopschafts) fixierbar ist, wie er beispielsweise in dem eingangs genannten Stand der Technik gemäß der US 7,413,543 B2 offenbart wird.

Erfindungsgemäß hat die Hülse 1 einen zylindrischen Hülsenkörper 1 a, der an seinem distalen axialen Endabschnitt oder Stirnseite eine sich radial einwärts erstreckende Halterung bzw. einen im Wesentliche teilkreisförmigen (in Draufsicht dreieckigen) Anschlusssockel 2 u.a. für einen Arbeitskanal hat, wobei die Halterung 2 vorliegend einstückig mit dem Hülsenkörper 1 a vorzugsweise aus einem Kunststoffmaterial ausgebildet (spritzgegossen) ist.

In der Halterung 2 ist dabei eine Anzahl von Durchgangsbohrungen 4-8 ausgearbeitet. Insbesondere ist eine zentrale Öffnung 6 vorgesehen, an die der nicht weiter dargestellte Arbeitskanal angeschlossen sowie daran gehalten wird, sowie eine dezentrale Öffnung 4 vorgesehen, welche als Durchtrittsöffnung für eine Optik dient, was nachfolgend im Einzelnen noch beschrieben wird. Des Weiteren ist eine kleindurchmessrige Fluidaustrittsöffnung (Spülöffnung) 8 in der Halterung 2 ausgeformt, an die ein Spülflüssigkeitskanal anschließbar ist.

Wie aus der Fig. 1 zu entnehmen ist, wird die distale stirnseitige Öffnung der Hülse 1 nur in etwa über einen Viertelkreis von der Halterung 2 abgedeckt. Der übrige Abschnitt der distalen Stirnseite (entspricht folglich in etwa einem Dreiviertelkreis) der Hülse 1 ist hingegen offen. An dieser Stelle sei darauf hingewiesen, dass die Verhältnisse "offen" zu "geschlossen" vom genannten Beispiel auch abweichen können. Sollte beispielsweise kein Spülkanal vorgesehen sein, könnte die Halterung 2 auch kleiner dimensioniert sein.

In dem offenen Kreisabschnitt (vorliegend Dreiviertelkreis) ist axial hinter der distalen Stirnseite und damit axial zur Halterung 2 zurückversetzt ein Wärmetauscher angeordnet, u.a. bestehend aus einer Kühlkammer 10 etwa in der Form, wie sie in der Fig. 2 (gemäß dem Stand der Technik) gezeigt ist. D.h. auch die Kühlkammer 10 gemäß der vorliegenden Erfindung hat im Grundriss eine Bananen- oder Sichelform (kleiner als der offene Dreiviertelkreis) mit einer zentralen, sich axial erstreckenden, sowie im Grundriss teilkreisförmigen Ausbuchtung, welche die zentrale Öffnung 6 in der Halterung 2 umgreift. Im Gegensatz zum Stand der Technik ist die erfindungsgemäße Kühlkammer 10 jedoch nicht als zur Hülse 1 separates Bauteil (Funktionseinheit) vorgesehen sondern einstückig mit der Hülse 1, vorzugsweise in einem einzigen Herstellungsschritt (Spritzgussverfahren) sowie aus dem gleichen Material wie die Hülse 1 ausgeformt. Aufgrund dieser einstückigen Ausgestaltung bildet die Halterung 2 sowie der Wärmetauscher zusammen mit der Hülse 1 ein gemeinsames Bauteil, wobei sich die Halterung 2 entlang ihres gesamten Umfangs am Wärmetauscher (an der Wandung der Kühlkammer 10) dichtend abstützt.

Axial hinter der Kühlkammer 10 bzw. hinter dem Wärmetauscher befindet sind ein erstes elektronisches Bauteil 12 vorliegend in Form einer Leiterplatte oder Platine, auf der sich eine Anzahl elektronischer Komponenten wie u.a. ein lichtempfindlicher Sensor (z.B. CMOS-Chip) 13 sowie ggf. eine Speichereinheit oder ein Interface zur elektrischen Datenübermittlung befindet. Des Weiteren ist ein Linsensystem 14 oberhalb des lichtempfindlichen Sensors 13 auf der Platine 12 platziert und ausgerichtet. Die Platine 12 ist mit Eingriffsmitteln (nicht weiter gezeigt) versehen, mittels denen die Platine 12 im Hülsenkörper 1a fixierbar ist. Insbesondere hat der Hülsenkörper 1a in seinem proximalen Endabschnitt entsprechend ausgebildete Rastelemente, die so angeordnet sind, dass die Platine 12 nach deren Verrastung im Hülsenkörper 1a dessen stirnseitigen proximalen Abschluss/Verschluss, gegenüberliegend zur distalen Stirnseite der Hülse 1, darstellt. Schließlich ist auf der ersten Platine 12 noch eine Anzahl von elektrischen Anschlüssen angeordnet, über welche die Platine 12 u.a. an elektrische Leitungen im nicht gezeigten Endoskopschaft anschließbar ist.

Der lichtempfindliche Sensor 13 sowie das darüber fixierte Linsensystem 14 sind so auf der ersten Platine 12 platziert, dass sie sich im eingebautem Zustand der ersten Platine 12 hinter der dezentralen Öffnung 4 in der Halterung 2 anordnen. Die axiale Länge der Kühlkammer 10 ist zudem in Abhängigkeit der Bauhöhe des Linsensystems 14 so bemessen, dass sich das Linsensystem 14 unmittelbar an die dezentrale Öffnung 4 anschließt oder durch diese sogar axial nach vorn vorragt. Die dezentrale Öffnung 4 hat hierbei einen Durchmesser, der größer ist als der Außendurchmesser des Linsensystems 14, sodass das Linsensystem 14 nicht mit dem Öffnungsrand in Kontakt kommt. Die dezentrale Öffnung 4 ist vorzugsweise mit einer lichtdurchlässigen Scheibe 16 (aus Plexiglas, Glas, Acryl, etc.) an der äußeren Seite der Halterung 2 dichtend verschlossen, um das Linsensystem 14 gegen Verschmutzung zu schützen.

Ferner ist eine Beleuchtung in Form einer zweiten Leiterplatte oder Platine 18 vorgesehen, auf der eine Anzahl von Leuchtmitteln, beispielsweise LED's oder Glühbirnen 20 montiert und elektrisch miteinander verbunden sind. Die zweite Leiterplatte18 hat zudem elektrische Anschlüsse zur Stromversorgung der Leuchtmittel 20, die mit Anschlüssen auf der ersten Platine 12 in elektrischen Kontakt bringbar sind. Die zweite Leiterplatte 18 hat zudem eine Grundrissform, welche dem Dreiviertelkreis und damit der Randkontur der Halterung 2 innerhalb der Hülse 1 entspricht (also in diesem Fall größer ist, als die Stirnseite der Kühlkammer 10), derart, dass die zweite Leiterplatte 18 von der distalen Stirnseite her in die Hülse 1 (in den Dreiviertelkreis) eingesetzt werden kann. Schließlich kann auch die zweite Leiterplatte 18 mittels einer transparenten Füllmasse und/oder einer lichtdurchlässigen Scheibe 22 genannten Materials abgedeckt werden, die an der Stirnseite der Hülse 2 oder unmittelbar an der zweiten Leiterplatte 18 fixiert wird.

Innerhalb der Kühlkammer 10 ist ein Kühlmittel-Strömungspfad 11 ausgebildet, der gegenüber der zweiten Leiterplatte 18 getrennt bzw. fluiddicht abgeschlossen ist, sodass kein Kühlmittel unmittelbar die zweite Leiterplatte 18 erreichen/berühren kann. Der Strömungspfad ist über Ein- und Auslässe in der Kühlkammer 10 an nicht gezeigte Zu- und Abfuhrleitung im Endoskopschaft angeschlossen. Die fluiddichte Trennung der Kühlkammer 10 / des Strömungspfads 11 und der zweiten Leiterplatte 18 kann über unterschiedliche konstruktive Maßnahmen erfolgen:
Gemäß einer Variante ist es vorgesehen, zwischen der zweiten Leiterplatte 18 und der Kühlkammer 10 eine Trennwand 24 in die Hülse 1 einzusetzen/ einzukleben/einzugießen, deren Umfangsform der Form der Kühlkammer 10 entspricht (also kleiner ist als die zweite Platine 18), sodass die Trennwand 24 ausschließlich die Kühlkammer 10 abdeckt. Wie aus der Fig. 1 zu entnehmen ist, sind auch an der zweiten Leiterplatte 18 eine Anzahl von elektrischen Kontakten oder Anschlüssen angeordnet, die am Wärmetauscher (an der Kühlkammer 10) vorgeführt sind, um mit entsprechenden Kontakten an der ersten Leiterplatte 12 in Kontakt zu kommen. Da die Trennwand 24 so geformt ist, dass sie nur die Kühlkammer 10 dichtend abdeckt, also eine vorliegend in etwa 0.3 mm dicke Abschlussplatte der Kühlkammer 10 ist, muss diese nicht mit Ausnehmungen oder Durchgangsbohrungen beispielsweise für die Anschlüsse der zweiten Leiterplatte 18 versehen sein.

Die Trennwand 24 besteht vorzugsweise aus einer (flexiblen) Membran oder Folie. Diese Folie oder Membran ist an der offenen Stirnseite der Kühlkammer 10 dichtend fixiert, derart, dass sie mechanischen, zumindest jedoch thermischen Kontakt mit der nachträglich eingesetzten zweiten Leiterplatte 18 erhält. Die Trennwand 24 kann aus dem gleichen Material bestehen wie die Hülse 1 (also allgemein ein Kunststoffmaterial), sie kann aber auch aus einem beschichteten Gewebe oder einer Metall-/Aluminiumfolie bestehen. Auch andere Materialien sind denkbar, die fluiddicht mit dem Hülsenmaterial verbindbar sind und einen noch ausreichenden Wärmeübertragungskoeffizienten aufweisen.

Gemäß einer bevorzugten Variante der Erfindung (wie auch in der Fig. 1 gezeigt wird) ist es vorgesehen, innerhalb der Kühlkammer 10 ein Kanalsystem anzuordnen/auszubilden mit einer geschlossenen Kanal-/Rohrwand 11, durch welches Kühlmittel hindurchgeführt wird. Dieses Kanalsystem nach dem Prinzip eines bekannten Wärmetauschers hat einen vorzugsweise geschleiften Längsabschnitt zur Streckenverlängerung, der unmittelbar entlang der Trennwand 24 führt und der folglich über die Trennwand 24 thermischen Kontakt mit der zweiten Leiterplatte 18 hat. In diesem Fall dient die Trennwand 24 dazu, das Kanalsystem 11 axial abzudichten/zu verschließen.

Schließlich besteht auch die Möglichkeit, das Kanalsystem 11 gemäß der Fig. 1 durch ein externes Rohr zu bilden, das in die Kühlkammer 10 eingesetzt und mit dem Ein- und Auslass des Wärmetauschers verbunden ist. Auch in diesem Fall kann eine Trennwand 24 gemäß der Fig. 1 zusätzlich vorgesehen sein.

Für die zweite Leiterplatte 18 ist es notwendig, dass thermische Energie, welche durch die Leuchtmittel 20 erzeugt wird, mit gutem Wirkungsgrad an das Kühlmittel abgegeben wird. Zu diesem Zweck verwendet die Erfindung vorzugsweise Platinen nach der IMS (insulated metallic substrate) - Technologie bestehend aus einer elektrisch leitenden Schicht zur Herstellung von Leiterbahnen, einer elektrisch isolierenden Zwischenschicht und einer Trägerlage/Substrat vorzugsweise einer Aluminiumplatte, die miteinander zu einer Verbund-Leiterplatte verklebt sind. Grundsätzlich besteht aber auch die Möglichkeit der Verwendung einer konventionellen Platine, wie sie in der Fachwelt auch als FR4-Platine bekannt ist.

Die Montage des erfindungsgemäßen Endoskopkopfs lässt sich wie Folgt beschreiben:
Zunächst sind in der Hülse 1 sämtliche Versorgungskanäle wie Spülkanal, Arbeitskanal etc. bereits ausgebildet und mit den entsprechenden Öffnungen 6, 8 in der sockelförmigen Halterung 2 einstückig gekoppelt. Demzufolge wird als erstes die Trennwand 24 von der axialen, distalen Außenseite der Hülse 1 her in die im Wesentlichen dreiviertelkreisförmige Öffnung der Hülse 1 eingeführt und auf/in der axialen Stirnseite der Kühlkammer 10 passgenau verklebt/vergossen, sodass hierdurch ein in sich fluiddicht geschlossener Wärmetauscher entsteht.

Wie vorstehend bereits ausgeführt wurde, ist die Trennwand 24 im Wesentlichen, vorzugsweise genau der Kontur der Kühlkammer 10 angepasst, sodass nur diese von der Trennwand 10 dichtend abgedeckt wird und daher axiale Durchgänge innerhalb der Hülse 1 für elektrische Kontakte oder Kabel frei bleiben.

Hierauf wird ebenfalls von der axialen, distalen Außenseite der Hülse 1 her die zweite Platine (als vormontierte Einheit) 18 in die vorzugsweise dreiviertelkreisförmige Öffnung der Hülse 1 eingesetzt und dann in der Hülse 1 fixiert (verklebt und/oder verklemmt). Vorzugsweise liegt hierbei die zweite Platine 18 rückseitig auf der Trennwand 24 teilweise auf, in jenem Platinenabschnitt, der sich mit der Trennwand 24 überlappt.

Schließlich kommt die lichtdurchlässige Vergussmasse in die dreiviertelkreisförmige Öffnung und optional die Abdeckscheibe 22 über die zweite Platine 18 sowie die Scheibe 16 über die Öffnung 4 die jeweils fluiddicht in/auf der Hülse 1 verklebt werden. In dieser Montageposition liegt die zweite Platine oder Leiterplatte 18 rückseitig an der Trennwand 24 an, welche den Innenraum der Kühlkammer 10 zur zweiten Leiterplatte 18 hin fluiddicht verschließt. Zusätzlich kann zwischen der zweiten Leiterplatte 18 und der Trennwand (Membran/Folie) 24 ein vorzugsweise klebriger Füllstoff angeordnet sein, der die Wärmeübertragung zwischen der zweiten Leiterplatte 18 und der Trennwand 24 noch verbessert. Die elektrischen Kontakte der zweiten Leiterplatte 18 führen dabei an der Kühlkammer 10 und der Trennwand 24 vorbei und ragen proximal über die Kühlkammer axial vor.

Abschließend wird die erste Leiterplatte (als vormontierte Einheit) 12 in den Hülsenkörper 1 a von der proximalen Stirnseite her eingesetzt, wobei sich die elektrischen Kontakte zur zweiten Leiterplatte 18 vorzugsweise selbsttätig schließen. Die Ausrichtung der ersten Leiterplatte 12 erfolgt mit dem Einsetzvorgang aufgrund der vorhandenen Rastmittel zwangsläufig so, dass sich die Optik bestehend aus lichtempfindlichem Sensor 13 und Linsensystem 14 genau unterhalb der entsprechenden dezentralen Öffnung 4 in der Halterung 2 anordnen, wobei die Ausrichtung der Optik ebenfalls ausschließlich durch die erste Leiterplatte 12 sowie deren korrekte Montagelage erfolgt. Die Optik selbst hat keinen direkten Kontakt mit der Hülse 1. Mit dem Einrasten der ersten Platine 12 in die Hülse 1 schiebt sich das Linsensystem 14 durch die entsprechende dezentrale Öffnung 4 hindurch, sodass eine freie Sicht nach vorn und ggf. radial zur Seite der Hülse 1 ermöglicht wird.

Abschließend sei die vorliegende Erfindung nochmals wie Folgt zusammengefasst:
Offenbart wird ein Endoskop mit flexiblem Endoskopschaft, an dessen distalem Ende ein Endoskopkopf angeordnet ist bestehend aus einer an beiden Stirnseiten offenen Hülse, in der Funktionseinheiten, insbesondere eine Optik, ein Arbeitskanal, eine Beleuchtung und eine Kühlung für die Beleuchtung untergebracht sind.

Erfindungsgemäß umfasst die Kühlung einen in der Hülse einstückig mit der Hülse ausgeformten Wärmetauscher, der eine geschlossene Kühlkammer zur Erzeugung einer Kühlmittelströmung ausbildet. Die Beleuchtung in Form einer Leuchtmittel tragenden externen Leiterplatte ist so platziert, dass sie an der distalen Stirnwand des Wärmetauschers anliegt (mechanisch aufliegt oder nur geringfügigen Abstand hat), um eine thermische Energie über die Stirnwand des Wärmetauschers in die Kühlmittelströmung abzugeben.

## Patentansprüche

1. Endoskop mit flexiblem Endoskopschaft, an dessen distalem Ende ein Endoskopkopf angeordnet ist bestehend aus einer an beiden Stirnseiten offenen Hülse (1), in der eine Optik, ein Arbeitskanal, eine Beleuchtung und eine Kühlungseinrichtung zumindest für die Beleuchtung untergebracht sind, wobei die Kühlungseinrichtung einen in der Hülse (1) stoffeinstückig mit dieser ausgeformten Wärmetauscher aufweist, der eine zusammen mit der Hülse (1) ausgebildete Kühlkammer (10) zur Erzeugung einer Kühlmittelströmung umfasst, wobei die Beleuchtung in Form einer Leuchtmittel tragenden externen Leiterplatte (18) an einer die Kühlkammer (10) fluiddicht verschließenden distalen Stirnwand (24) des zumindest abschnittsweise an ihr entlang verlaufende Kanalschleifen aufweisenden Wärmetauschers so anliegt, um eine thermische Energie über die Stirnwand (24) des Wärmetauschers in die Kühlmittelströmung abzugeben, wobei die Kühlkammer (10) einen nach Außerhalb der Kühlkammer (10) abgeschlossenen Kühlmittelpfad (11) in Form eines in der Kühlkammer (10) definierten Kanals ausbildet, der zumindest abschnittsweise entlang der distalen Stirnwand des Wärmetauschers verläuft und wobei in der Hülse (1) im axialen Bereich ihrer distalen Stirnseite eine radial nach innen ragende, sowie stoffeinstückig am Wärmetauscher sich abstützende Halterung (2) für den Arbeitskanal und vorzugsweise zusätzlich für einen Spülkanal angeformt ist, die ferner eine axiale Durchgangsöffnung (4) für die Optik aufweist **dadurch gekennzeichnet, dass** die Optik aus einer einen lichtempfindlichen Sensorchip (13) tragenden weiteren Leiterplatte (12) besteht, die sich proximal der Kühlkammer (10) befindet und mit Eingriffsmitteln versehen ist, mittels denen die weitere Leiterplatte (12) im Hülsenkörper (1 a) fixierbar ist, und auf der oberhalb des Sensorchips (13) ein Linsensystem (14) am Sensorchip (13) montiert ist, derart, dass das Linsensystem (14) kontaktlos zur Hülse (1) ausschließlich über die den Sensorchip (13) tragende weitere Leiterplatte (12) gehalten wird und frei durch die Durchgangsöffnung (4) in der Halterung (2) für den Arbeitskanal geführt ist.

2. Endoskop nach Anspruch 1, **dadurch gekennzeichnet, dass** der Kanal (11) durch eine Rohrleitung gebildet ist, die separat gefertigt und in die Kühlkammer (10) eingesetzt ist oder die einstückig mit der Kühlkammer (10) und damit mit der Hülse (1) ausgeformt ist.

3. Endoskop nach einem der Ansprüche 1 bis 2, **gekennzeichnet durch** eine externe separate Trennwand, welche die distale Stirnwand (24) des Wärmetauschers zum stirnseitig dichtenden Verschließen und fluiddichten Trennen der Kühlkammer (10) von der Leuchtmittel tragenden externen Leiterplatte (18) sowie zur Gewährleistung einer Übertragung an thermischer Energie von den Leuchtmitteln in das Kühlmittel ausbildet.

4. Endoskop nach Anspruch 3, **dadurch gekennzeichnet, dass** die Trennwand (24) unterschiedlich oder unabhängig zu der Form der die Leuchtmittel tragenden externen Leiterplatte (18) an die Form der Kühlkammer (10) angepasst ist, um ausschließlich die Kühlkammer (10) axial abzudecken.

5. Endoskop nach einem der Ansprüche 1 bis 4, **dadurch gekennzeichnet, dass** die Stirnwand (24) des Wärmetauschers eine Membran oder Folie ist, die mit der Hülse (1) fluiddicht verklebt und/oder vergossen ist.

6. Endoskop nach einem der Ansprüche 1 bis 5, **gekennzeichnet durch** einen Kühlmittelein- und -auslass, die in einem Winkelabstand zueinander an der proximalen Stirnseite des Wärmetauschers ausgebildet sind.

7. Endoskop nach Anspruch 1, **dadurch gekennzeichnet, dass** die den Sensorchip (13) tragende weitere Leiterplatte (12) quer zur Axialrichtung der Hülse (1), vorzugsweise parallel zur distalen Stirnseite der Hülse (1) montiert ist und den proximalen stirnseitigen Abschluss der Hülse (1) bildet.

8. Endoskop nach Anspruch 7, **dadurch gekennzeichnet, dass** die den Sensorchip (13) tragende weitere Leitplatte (12) in thermischem Kontakt mit der Kühlkammer (10) steht, vorzugsweise an dieser stirnseitig anliegt, um die darauf befindlichen elektrischen/optischen Bauteile zu kühlen.

9. Endoskop nach Anspruch 8, **dadurch gekennzeichnet, dass** die Axiallänge der Kühlkammer (10) an die Optik derart angepasst ist, dass das Linsensystem (14) in montiertem Zustand in der Durchgangsöffnung (4) der Halterung (2) endet oder axial über die Durchgangsöffnung (4) in distaler Richtung vorragt.

10. Endoskop nach einem der Ansprüche 1 bis 9, **dadurch gekennzeichnet, dass** der axiale Abschnitt zwischen der Leuchtmittel tragenden, externen Leiterplatte (18) und der distalen Stirnseite der Hülse (1) mit einer lichtdurchlässigen Vergussmasse aufgefüllt ist, welche stirnseitig entweder mittels einer transparenten Platte (22) abgedeckt ist oder welche zusammen mit der distalen Vorderseite der Halterung (2) für den Arbeitskanal die axiale Stirnseite der Hülse (1) bildet.

11. Endoskop nach einem der Ansprüche 1 bis 10, **dadurch gekennzeichnet, dass** die Hülse (1) und die Kühlkammer (10) als ein einziges, integrales Kunststoff-Spritzgussteil ausgebildet sind.

12. Endoskop nach einem der Ansprüche 1 bis 11, **dadurch gekennzeichnet, dass** die Hülse (1) und die Kühlkammer (10) vorzugsweise mit Ausnahme der distalen Stirnwand der Kühlkammer (10) als ein einziges, integrales Kunststoff-Spritzgussteil ausgebildet sind.

## Claims

1. An endoscope comprising a flexible endoscope shaft at the distal end of which an endoscope head is arranged consisting of a sleeve (1) open at both end faces in which an optical system, a working channel, an illumination and a cooling means at least for the illumination are accommodated, wherein the cooling means has a heat exchanger formed in the sleeve (1) integrally with the latter and comprising a cooling chamber formed together with the sleeve (1) for generating a coolant flow, wherein
the illumination in the form of an external conductor plate (18) supporting an illumination means is adjacent to a distal end wall (24) of the heat exchanger, which extends at least in portions along the distal end wall and having channel loops, closing the cooling chamber in a fluid-tight manner so as to discharge thermal energy via the end wall (24) of the heat exchanger to the coolant flow, wherein the cooling chamber (10) forms a coolant path (11) closed to the outside of the cooling chamber (10) in the form of a channel defined in the cooling chamber (10) which extends at least in portions along the distal end wall of the heat exchanger,
and wherein a radially inwardly projecting support (2) is integrally supported on the heat exchanger for the working channel and preferably in addition for a flushing channel is formed in the sleeve (1) in the axial area of its distal end face, the
support (2) further including an axial through-opening (4) for the optical system, **characterized in that**
the optical system consists of an additional conductor plate (12) supporting a light-sensitive sensor chip (13) which is arranged proximally to the cooling chamber (10) and which is provided with engaging means by means of which the additional conductor plate (12) is adapted to be fixed in the sleeve body (1a), and on which a lens system (14) is mounted above the sensor chip (13) at the sensor chip (13) such that the lens system (14) is held without contacting the sleeve (1) exclusively via the additional conductor plate (12) supporting the sensor chip (13) and is guided freely through the through-opening (4) in the support (2) for the working channel.

2. The endoscope according to claim 1, **characterized in that** the channel (11) is formed by a tubing which is manufactured separately and is inserted in the cooling chamber (10) or which is formed integrally with the cooling chamber (10) and thus with the sleeve (1).

3. The endoscope according to one of the claims 1 to 2, **characterized by** an external separate partition wall which forms the distal end wall (24) of the heat exchanger for sealing at the end face and separating the cooling chamber (10) in a fluid-tight manner from the external conductor plate (18) supporting the illumination means as well as for ensuring transfer of thermal energy from the illumination means to the coolant.

4. The endoscope according to claim 3, **characterized in that** the partition wall (24) is adapted, differently from or independently of the shape of the external conductor plate (18) supporting the illumination means, to the shape of the cooling chamber (10) so as to axially cover exclusively the cooling chamber (10).

5. The endoscope according to one of the claims 1 to 4, **characterized in that** the end wall (24) of the heat exchanger is a membrane or film that is glued and/or sealed with the sleeve (1) in a fluid-tight manner.

6. The endoscope according to one of the claims 1 to 5, **characterized by** a coolant inlet and outlet formed at an angular distance from each other at the proximal end face of the heat exchanger.

7. The endoscope according to claim 1, **characterized in that** the additional conductor plate (12) supporting the sensor chip (13) is mounted transversely to the axial direction of the sleeve (1), preferably in parallel to the distal end face of the sleeve (1) and constitutes the proximal end of the sleeve (1) at the end face.

8. The endoscope according to claim 7, **characterized in that** the additional conductor plate (12) supporting the sensor chip (13) is in thermal contact with the heat exchanger, preferably is adjacent to the latter at the end face so as to cool the electric/optical components provided thereon.

9. The endoscope according to claim 8, **characterized in that** the axial length of the cooling chamber (10) is adapted to the optical system in such way that the lens system (14) in the mounted state ends in the through-opening (4) of the support (2) or projects axially from the through-opening (4) in the distal direction.

10. The endoscope according to one of the claims 1 to 9, **characterized in that** the axial portion between the external conductor plate (18) supporting the illumination means and the distal end face of the sleeve (1) is filled with a translucent sealing compound which is covered at the end face either by a transparent plate (22) or which forms the axial end face of the sleeve (1) together with the distal front side of the support (2) for the working channel.

11. The endoscope according to one of the claims 1 to 10, **characterized in that** the sleeve (1) and the cooling chamber (10) are in the form of a single integral injection molded part made of plastic material.

12. The endoscope according to one of the claims 1 to 11, **characterized in that** the sleeve (1) and the cooling chamber (10) are in the form of a single integral injection molded part made of plastic material preferably with the exception of the distal end wall of the cooling chamber (10).

## Revendications

1. Endoscope avec une tige d'endoscope flexible, au niveau de l'extrémité distale de laquelle est disposée une tête d'endoscope, constitué d'un manchon (1) ouvert au niveau des deux faces frontales, dans lequel sont logés une optique, un canal de travail, un éclairage et un dispositif de refroidissement, au moins pour l'éclairage, le dispositif de refroidissement comprenant un échangeur thermique formé dans le manchon (1) d'une seule pièce avec celui-ci, qui comprend une chambre de refroidissement (10) réalisée conjointement avec le manchon (1), pour la production d'un écoulement de fluide de refroidissement, l'éclairage s'appuyant, sous la forme d'un circuit imprimé externe (18) supportant un moyen d'éclairage, contre une paroi frontale (24), obturant la chambre de refroidissement (10) de manière étanche aux fluides, de l'échangeur thermique comprenant au moins partiellement des boucles de canaux s'étendant le long de celui-ci, de façon à distribuer une énergie thermique par l'intermédiaire de la paroi frontale (24) de l'échangeur thermique dans l'écoulement de fluide de refroidissement, la chambre de refroidissement (10) formant un chemin de fluide de refroidissement (11) obturé vers l'extérieur de la chambre de refroidissement (10), sous la forme d'un canal défini dans la chambre de refroidissement (10), qui s'étend au moins partiellement le long de la paroi frontale distale de l'échangeur thermique et, dans le manchon (1), dans la zone axiale de sa face frontale distale, est moulé un support (2), dépassant radialement vers l'intérieur et s'appuyant d'une seule pièce contre l'échangeur thermique, pour le canal de travail et de préférence en outre pour un canal de rinçage, qui comprend en outre une ouverture de passage axiale (4) pour l'optique,
**caractérisé en ce que** l'optique est constituée d'un autre circuit imprimé (12) supportant une puce de capteur photosensible, qui est proximal par rapport à la chambre de refroidissement (10) et qui est munie de moyens d'emboîtement, au moyen desquels l'autre circuit imprimé (12) peut être fixé dans le corps de manchon (1 a), et sur lequel, au-dessus de la puce de capteur (13), est monté un système de lentilles (14) sur la puce de capteur (13), de façon à ce que le système de lentilles (14) soit maintenu sans contact avec le manchon (1) exclusivement par l'intermédiaire de l'autre circuit imprimé (12) supportant la puce de capteur (13) et soit guidé librement à travers l'ouverture de passage (4) dans le support (2) pour le canal de travail.

2. Endoscope selon la revendication 1, **caractérisé en ce que** le canal (11) est constitué d'une conduite tubulaire qui est réalisée séparément et qui est insérée dans la chambre de refroidissement (10) ou qui est formée d'une seule pièce avec la chambre de refroidissement (10) et donc avec le manchon (1).

3. Endoscope selon l'une des revendications 1 à 2, **caractérisé par** une cloison distincte externe qui forme la paroi frontale distale (24) de l'échangeur thermique pour l'obturation étanche côté frontal et la séparation étanche aux fluides de la chambre de refroidissement (10) par rapport au circuit imprimé externe (18) supportant les moyens d'éclairage ainsi que pour garantir une transmission d'énergie thermique des moyens d'éclairage vers le fluide de refroidissement.

4. Endoscope selon la revendication 3, **caractérisé en ce que** la cloison (24) est adaptée, de manière différente ou en fonction de la forme du circuit imprimé externe (18) supportant les moyens d'éclairage, à la forme de la chambre de refroidissement (10) afin de recouvrir axialement exclusivement la chambre de refroidissement (10).

5. Endoscope selon l'une des revendications 1 à 4, **caractérisé en ce que** la paroi frontale (24) de l'échangeur thermique est une membrane ou un film qui est collé et/ou moulé de manière étanche aux fluides avec le manchon (1).

6. Endoscope selon l'une des revendications 1 à 5, **caractérisé par** une entrée et une sortie de fluide de refroidissement, qui sont réalisées avec une distance angulaire entre elles au niveau de la face frontale proximale de l'échangeur thermique.

7. Endoscope selon la revendication 1, **caractérisé en ce que** l'autre circuit imprimé (12) supportant la puce de capteur (13) est monté transversalement par rapport à la direction axiale du manchon (1), de préférence parallèlement à la face frontale distale du manchon (1) et constitue la terminaison frontale proximale du manchon (1).

8. Endoscope selon la revendication 7, **caractérisé en ce que** l'autre circuit imprimé (12) supportant la puce de capteur (13) est en contact thermique avec la chambre de refroidissement (10), de préférence s'appuie frontalement contre celle-ci, afin de refroidir les composants électriques/optiques se trouvant sur celle-ci.

9. Endoscope selon la revendication 8, **caractérisé en ce que** la longueur axiale de la chambre de refroidissement (10) est adaptée à l'optique de façon à ce que le système de lentilles (14) se termine, dans l'état monté, dans l'ouverture de passage (4) du support (2) ou dépasse axialement de l'ouverture de passage (4) dans la direction distale.

10. Endoscope selon l'une des revendications 1 à 9, **caractérisé en ce que** la portion axiale entre le circuit imprimé externe (18) supportant les moyens d'éclairage et la face frontale distale du manchon (1), est remplie d'une masse de scellement transparente qui est soit recouverte, côté frontal, par une plaque transparente (22), soit forme, conjointement avec la face avant distale du support (2) pour le canal de travail, la face frontale axiale du manchon (1).

11. Endoscope selon l'une des revendications 1 à 10, **caractérisé en ce que** le manchon (1) et la chambre de refroidissement (10) sont réalisés sous la forme d'une seule et unique pièce en matière plastique injectée.

12. Endoscope selon l'une des revendications 1 à 11, **caractérisé en ce que** le manchon (1) et la chambre de refroidissement (10) sont réalisés de préférence, à l'exception de la paroi frontale distale de la chambre de refroidissement (10), sous la forme d'une seule et unique pièce en matière plastique injectée.
